(19) Europäisches Patentamt
European Patent Office
Office européen des brevets

(11) **EP 3 118 332 B1**

(12) **EUROPEAN PATENT SPECIFICATION**

(45) Date of publication and mention
of the grant of the patent:
**06.06.2018 Bulletin 2018/23**

(51) Int Cl.:
*C12Q 1/68* *(2018.01)*    *G01N 33/53* *(2006.01)*

(21) Application number: **16179737.8**

(22) Date of filing: **15.07.2016**

(54) **DNA METHYLATION DETECTION**

DNA-METHYLIERUNGSNACHWEIS

DÉTECTION DE LA MÉTHYLATION D'ADN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **15.07.2015 US 201562192987 P**

(43) Date of publication of application:
**18.01.2017 Bulletin 2017/03**

(73) Proprietor: **Helios Bioelectronics Inc.
Hsinchu County 30261 (TW)**

(72) Inventors:
• **Chan, Hardy Wai-Hong
Redwood City, CA 94065 (US)**
• **Yang, Yuh-Shyong
300 Hsinchu (TW)**
• **Chen, Wen-Yih
320 Taoyuan City (TW)**
• **Fu, Chang-Wei
Pingtung County 928 (TW)**

(74) Representative: **Fuchs Patentanwälte
Partnerschaft mbB
Westhafenplatz 1
60327 Frankfurt am Main (DE)**

(56) References cited:
**EP-A1- 2 143 806    US-A1- 2007 231 790**

• **ANRAN GAO ET AL: "Enhanced Sensing of Nucleic Acids with Silicon Nanowire Field Effect Transistor Biosensors", NANO LETTERS, vol. 12, no. 10, 10 October 2012 (2012-10-10), pages 5262-5268, XP055325402, US ISSN: 1530-6984, DOI: 10.1021/nl302476h**
• **YASUHIDE OHNO ET AL: "Label-Free Biosensors Based on Aptamer-Modified Graphene Field-Effect Transistors", JOURNAL OF THE AMERICAN CHEMICAL SOCIETY, vol. 132, no. 51, 29 December 2010 (2010-12-29), pages 18012-18013, XP055100068, ISSN: 0002-7863, DOI: 10.1021/ja108127r**
• **MAKI ET AL: "Nanowire-transistor based ultra-sensitive DNA methylation detection", BIOSENSORS AND BIOELECTRONICS, ELSEVIER BV, NL, vol. 23, no. 6, 20 December 2007 (2007-12-20), pages 780-787, XP022396668, ISSN: 0956-5663, DOI: 10.1016/J.BIOS.2007.08.017**
• **RYOJI KURITA ET AL: "DNA Methylation Analysis Triggered by Bulge Specific Immuno-Recognition", ANALYTICAL CHEMISTRY, vol. 84, no. 17, 4 September 2012 (2012-09-04), pages 7533-7538, XP055213627, ISSN: 0003-2700, DOI: 10.1021/ac301702y**

**Description**

**FIELD OF THE INVENTION**

This invention relates to a molecular detection technique. More particularly, the invention relates to DNA methylation detection.

**BACKGROUND OF THE INVENTION**

Molecular detection plays an important role in clinical diagnosis and molecular biology research. For example, DNA methylation detection is regarded as a key detection in several applications. The methylation of the 5' carbon of cytosine (also known as methylated cytosine or 5-methylcytosine) in DNA is an epigenetic modification that regulates gene expression and plays crucial roles in embryonic development, and is thought to be involved in cancer, genomic imprinting, cellular differentiation, and Alzheimer's disease (Kurita, Ryoji, and Osamu Niwa. "DNA methylation analysis triggered by bulge specific immuno-recognition." Analytical chemistry 84.17 (2012): 7533-7538).

Several systems have been developed to perform DNA methylation detection for detecting and/or identifying the number and/or position of 5-methylcytosine in a DNA molecule. The major approach for DNA methylation detection is a bisulfite conversion method (Lister, Ryan, et al. "Human DNA methylomes at base resolution show widespread epigenomic differences." Nature 462.7271 (2009): 315-322). In the method, a DNA molecule to be tested is treated with bisulfite to convert cytosine nucleotides to uridine nucleotides, leaving 5-methylcytosine nucleotides unconverted. The converted uridine nucleotides are further converted to thymine nucleotides in the subsequent replication of polymerase chain reaction. On the other hand, the unconverted 5-methylcytosine nucleotides remain cytosine nucleotides in the replication. For distinguishing the converted thymine nucleotides from unconverted cytosine nucleotides, a gene sequencing or microarray process is applied. For example, the whole genome shotgun bisulfite sequencing (WGSGS) is utilized to analyze the whole gene sequence after bisulfite conversion, and the methylation pattern in a specific situation can be obtained with single-base resolution. However, the sequencing is laborious and costly. In order to remedy the defects, a method of reduced representation bisulfite sequencing (RRBS) coupled with restriction enzymes was developed to eliminate the unnecessary fragments, but the cost is still unsatisfactory. Another approach is using the microarray process, which is able to analyze the methylation pattern of a specific fragment in a fast and high-throughput manner, and is widely applied in drug screening. Nevertheless, the microarray process fails to determine the number and position of the 5-methylcytosine nucleotides.

An isoschizomer method for DNA methylation detection has also been developed. Isoschizomers are pairs of restriction enzymes specific to the same recognition site. One of the common isoschizomers is a methylation-sensitive enzyme that recognizes only unmethylated cytosine nucleotides and does not recognize methylated cytosine nucleotides. Another common isoschizomers is a methylation-dependent enzyme that recognizes only methylated cytosine nucleotides and does not recognize un-methylated cytosine nucleotides. By incorporating different kinds of isoschizomers, the 5-methylcytosine nucleotides can be detected. Unfortunately, application of the method is highly limited due to the inherent property of the isoschizomers, and only the 5-methylcytosine nucleotides located in the recognition site of a specific pair of isoschizomers can be detected.

Another approach for DNA methylation detection is an affinity method. Antibodies or proteins with specific affinity to the 5-methylcytosine nucleotides are utilized for capturing a fragment containing 5-methylcytosine nucleotides, and a gene sequencing or microarray process is applied subsequently to analyze the captured fragment. Examples of affinity methods are methylated DNA immunoprecipitation (MeDIP) using the antibodies (Weber, Michael, et al. "Chromosome-wide and promoter-specific analyses identify sites of differential DNA methylation in normal and transformed human cells." Nature genetics 37.8 (2005): 853-862) or methylated CGI recovery assay (MIRA) using methyl binding domain proteins (MBDs) (Cross, Sally H., et al. "Purification of CpG islands using a methylated DNA binding column." Nature genetics 6.3 (1994): 236-244; Kurita, Ryoji, and Osamu Niwa. "DNA methylation analysis triggered by bulge specific immuno-recognition." Analytical chemistry 84.17 (2012): 7533-7538). In the affinity method, the fragment is provided by a manner such as sonication and the single-stranded fragment is also provided if necessary. The antibody or protein is first used to capture the fragment containing 5-methylcytosine nucleotides. After being separated from the antibody or protein, the captured fragment is analyzed by gene sequencing or microarray. Because the sequencing or microarray process is needed, the defects resulting from the process as mentioned above cannot be avoided.

**SUMMARY OF THE INVENTION**

In order to improve DNA methylation detection sensitivity and accuracy, a quick and convenient detection method is provided.

The invention is to provide a method for detecting methylation of a target oligonucleotide molecule, comprising obtaining an electrical change occurring due to the binding of an electrically charged methylation detecting molecule and the target oligonucleotide molecule; wherein the electrically charged methylation detecting molecule has affinity to a methylated cytosine nucleotide, as defined in the appended claims.

The present invention is also to provide a kit for detecting methylation of a target oligonucleotide molecule comprising:

an electrically charged methylation detecting molecule having affinity to a methylated cytosine nucleotide; and

an electrical change detecting element for detecting an electrical change, as defined in the appended claims.

The present invention is described in detail in the following sections. Other characteristics, purposes and advantages of the present invention can be found in the detailed description and claims.

## BRIEF DESCRIPTION OF THE DRAWINGS

FIG. 1 shows a schematic drawing of a method for detecting methylation of a target oligonucleotide molecule in one embodiment of the invention.

FIG. 2 shows one preferred embodiment of the electrically neutral nucleotide according to the invention.

FIG. 3 shows $I_d$-$V_g$ curve of SEPT9 DNA probe 1 with SEPT9 methyl DNA target 1 (C5). "■" indicates the electrical signal detected in 10 mM bis-tris propane buffer; "●" indicates the electrical signal induced by SEPT9 methyl DNA target 1; "▲" indicates the electrical signal induced by 1 $\mu$g/mL anti-methylcytosine antibody.

FIG. 4 shows $I_d$-$V_g$ curve of SEPT9 DNA probe 1 with SEPT9 methyl DNA target 2 (C14). "■" indicates the electrical signal detected in 10 mM bis-tris propane buffer; "●" indicates the electrical signal induced by SEPT9 methyl DNA target 2; "▲" indicates the electrical signal induced by 1 $\mu$g/mL anti-methylcytosine antibody.

FIG. 5 shows $I_d$-$V_g$ curve of SEPT9 DNA probe 1 with SEPT9 methyl DNA target 3 (C26). "■" indicates the electrical signal detected in 10 mM bis-tris propane buffer; "●" indicates the electrical signal induced by SEPT9 methyl DNA target 3; "▲" indicates the electrical signal induced by 1 $\mu$g/mL anti-methylcytosine antibody.

FIG. 6 shows the threshold voltage shift of nanowire FET (NWFET) induced by target DNA (left column) and antibody (right column).

FIG. 7 shows the threshold voltage shift ratio of antibody to target DNA.

FIG. 8 shows $I_d$-$V_g$ curve of SEPT9 DNA probe 2 with SEPT9 methyl DNA target 6 (C2,16,32). "■" indicates the electrical signal detected in 10 mM Bis-Tris Propane buffer; "●" indicates the electrical signal induced by SEPT9 methyl DNA target 6; "▲" indicates the electrical signal induced by 0.25 $\mu$g/mL anti-methylcytosine antibody.

FIG. 9 shows the threshold voltage shift of NWFET induced by target DNA (left column) and antibody (right column). 1x means the target oligonucleotide molecule has one methylated cytosine nucleotide. 2x means the target oligonucleotide molecule has two methylated cytosine nucleotides. 3x means the target oligonucleotide molecule has three methylated cytosine nucleotides.

FIG. 10 shows the threshold voltage shift ratio of antibody to target oligonucleotide molecule.

FIG. 11 shows $I_d$-$V_g$ curve of SEPT9 DNA probe 2 with SEPT9 methyl DNA target 7.

FIG. 12 shows $I_d$-$V_g$ curve of partially neutral single-stranded oligonucleotide (N-DNA) probe binding with SEPT9 methyl DNA target 6 (C2, C16, C32) and antibody. "■" indicates the electrical signal detected in 10 mM bis-tris propane buffer; "●" indicates the electrical signal induced by SEPT9 methyl DNA target 6; "▲" indicates the electrical signal induced by 0.1 $\mu$g/mL anti-methylcytosine antibody.

## DETAILED DESCRIPTION OF THE INVENTION

The invention is to provide a method for detecting methylation of a target oligonucleotide molecule, comprising obtaining an electrical change occurring due to the binding of an electrically charged methylation detecting molecule and the target oligonucleotide molecule; wherein the electrically charged methylation detecting molecule has affinity to a methylated cytosine nucleotide, as defined in the appended claims.

As used herein, the term "an oligonucleotide" or "an oligonucleotide molecule" refers to an oligomer of nucleotide. The term "nucleotide" refers to an organic molecule composed of a nitrogenous base, a sugar, and one or more phosphate groups; preferably one phosphate group. The nitrogenous base includes a derivative of purine or pyrimidine. The purine includes substituted or unsubstituted adenine and substituted or unsubstituted guanine; the pyrimidine includes substituted or unsubstituted thymine, substituted or unsubstituted cytosine and substituted or unsubstituted uracil. The sugar is preferably a five-carbon sugar, more preferably substituted or unsubstituted ribose or substituted or unsubstituted deoxyribose. The phosphate groups form bonds with the 2, 3, or 5-carbon of the sugar; preferably, with the 5-carbon site. For forming the oligonucleotide, the sugar of one nucleotide is joined to the adjacent sugar by a phosphodiester bridge. Preferably, the oligonucleotide is DNA or RNA; more preferably, DNA.

As used herein, the term "a target oligonucleotide molecule" refers to a naturally occurring or artificial molecule. In another aspect, the target oligonucleotide molecule is purified or mixed with other contents. In one preferred embodiment of the invention, the methylation pattern of the target oligonucleotide molecule is different in a normal condition and in an abnormal condition, such as a disease. In another preferred embodiment of the invention, the methylation pattern of the target oligonucleotide molecule is different in different cell types.

As used herein, the term "methylation of a target oligonucleotide molecule" refers to methylated nucleotides in a target oligonucleotide molecule. Particularly, the methylated nucleotide refers to a methylated cytosine nucleotide. As used herein, the methylated cytosine nucleotide has a methyl substitution in the 5' carbon of cytosine, and is also known as 5-methylcytosine nucleotide. When the target oligonucleotide molecule comprises a methylated cytosine nucleotide, the target oligonucleotide molecule has a target sequence with at least one methylated cytosine nucleotide.

As used herein, the term "detecting methylation of a target oligonucleotide molecule" refers to discovering or determining a profile or pattern of methylation of a target oligonucleotide molecule, including but not limited to the position, number or percentage of methylated nucleotides in the target oligonucleotide molecule.

As used herein, the term "an electrically charged methylation detecting molecule" refers to a molecule that has affinity to a methylated cytosine nucleotide, and carries electrical charges. Preferably, the electrically charged methylation detecting molecule is a protein that specifically binds to a methylated cytosine nucleotide. In one preferred embodiment of the invention, the electrically charged methylation detecting molecule is selected from the group consisting of an anti-methylcytosine antibody, a methyl binding domain protein and a restriction enzyme. The anti-methylcytosine antibody is a monoclonal antibody or a polyclonal antibody. In one embodiment of the invention, the anti-methylcytosine antibody recognizes a methylated cytosine nucleotide in a single-stranded oligonucleotide molecule, and in another embodiment of the invention, the anti-methylcytosine antibody recognizes a methylated cytosine nucleotide in a double-stranded oligonucleotide molecule. In another aspect, in one embodiment of the invention, the methyl binding domain protein binds to a methylated cytosine nucleotide in a single-stranded oligonucleotide molecule, and in another embodiment of the invention, the methyl binding domain protein binds to a methylated cytosine nucleotide in a double-stranded oligonucleotide molecule. Examples of the methyl binding domain protein include methy-CpG-binding domain (MBD) 1, MBD2, MBD3, and MBD4 protein. In still another aspect, in one embodiment of the invention, the restriction enzyme recognizes a methylated cytosine nucleotide in a single-stranded oligonucleotide molecule, and in another embodiment of the invention, the restriction enzyme recognizes a methylated cytosine nucleotide in a double-stranded oligonucleotide molecule. Examples of the restriction enzymes include HpaII, MspI, SmaI, and XmaI.

According to the invention, if a methylated cytosine nucleotide is present in the target oligonucleotide molecule, the electrically charged methylation detecting molecule binds to the methylated cytosine nucleotide of the target oligonucleotide molecule. Because the electrically charged methylation detecting molecule carries electrical charges, an electrical change occurs due to the binding of the electrically charged methylation detecting molecule and the target oligonucleotide molecule by introducing the electrical charges of the electrically charged methylation detection molecule into a complex formed by the electrically charged methylation detection molecule and the target oligonucleotide molecule. On the other hand, if a methylated cytosine nucleotide is absent from the target oligonucleotide molecule, the electrically charged methylation detecting molecule fails to bind to the methylated cytosine nucleotide of the target oligonucleotide molecule. Therefore, the electrical environment is unchanged, and no electrical change occurs. By monitoring of the electrical change, the methylation of the target oligonucleotide molecule is detected, and the profile or pattern of methylation of the target oligonucleotide molecule is determined.

The electrical change according to the invention includes but is not limited to increase of electrical charges. The electrical change can be detected as an electrical signal. The electrical signal includes but is not limited to changes of electric conductivity, electric field, electric capacitance, electric current, electron, or electron hole. In one preferred embodiment of the invention, the electrical change is a threshold voltage shift change.

Preferably, the electrical change is detected by an electrical change detecting element. The electrical change detecting element is applied for detecting whether the electrical change occurs due to the binding of the electrically charged methylation detecting molecule and the target oligonucleotide molecule. Preferably, the electrical change detecting element is a field-effect transistor or a surface plasmon resonance.

In one preferred embodiment of the invention, the method for detecting methylation of the target oligonucleotide molecule comprises:

capturing a single strand of the target oligonucleotide molecule by a recognizing single-stranded oligonucleotide molecule to form a duplex according to base complementarity;

providing the electrically charged methylation detecting molecule; and

4

binding the duplex with the electrically charged methylation detecting molecule and detecting the electrical change due to the binding, as defined in the appended claims.

The target oligonucleotide molecule can be a single-stranded molecule or a double-stranded molecule. The manner of obtaining the single strand of the double-stranded target oligonucleotide molecule can be, for example, heating or changing ion strength of the environment of the double-stranded target oligonucleotide molecule.

As used herein, the term "a recognizing single-stranded oligonucleotide molecule" refers to a single-stranded oligonucleotide molecule able to form a duplex with the target oligonucleotide molecule according to base complementarity. In other words, the recognizing single-stranded oligonucleotide molecule acts as a probe to hybridize the target oligonucleotide molecule. The duplex preferably refers to a double-stranded structure, and one strand is the single strand of the target oligonucleotide molecule and the other strand is the recognizing single-stranded oligonucleotide molecule. Preferably, the recognizing single-stranded oligonucleotide molecule has a sequence matched to the target sequence; more preferably, has a sequence perfectly matched to the target sequence. By forming the duplex, the target oligonucleotide molecule can be captured from a mixture in a sample. The capturing step also refers to a purification step of specifically selecting the target oligonucleotide molecule and presenting the target oligonucleotide molecule in the duplex. Consequently, the electrically charged methylation detecting molecule binds to the duplex, especially the methylated cytosine nucleotide of the target nucleotide molecule in the duplex, and the electrical change occurring due to the binding is obtained.

The sample according to the invention is derived from a naturally occurring origin or derived from artificial manipulation. Preferably, the sample is derived from a naturally occurring origin such as an extract, body fluid, tissue biopsy, liquid biopsy, or cell culture. In another aspect, the sample is processed according to the reaction of detection. For example, the pH value or ion strength of the sample may be adjusted.

Preferably, the duplex formed by the single strand of the target oligonucleotide molecule and the recognizing single-stranded oligonucleotide molecule comprises a bulge and the methylated cytosine nucleotide is disposed in the bulge. The sequence of the recognizing single-stranded oligonucleotide molecule is preferably to be designed to form the bulge with the single strand of the target oligonucleotide molecule. The bulge allows the methylated cytosine nucleotide to be presented well to the electrically charged methylation detecting molecule in the three-dimensional structure of the duplex.

The recognizing single-stranded oligonucleotide molecule according to the invention may be presented in a solution or attached to a solid surface. Preferably, the recognizing single-stranded oligonucleotide molecule is attached to a solid surface or the recognizing single-stranded oligonucleotide molecule is spaced apart from the solid surface by a distance.

As used herein, the term "solid surface" refers to a solid support including but not limited to a polymer, paper, fabric, or glass. Preferably, the solid surface to be employed varies depending on the electrical change detecting element. For example, when the method adopts a field-effect transistor to detect the electrical change, the solid surface is a transistor surface of the field-effect transistor; when the method adopts a surface plasmon resonance, the solid surface is a metal surface of a surface plasmon resonance.

In a preferred embodiment of the invention, the material of the solid surface is silicon; preferably polycrystalline silicon or single crystalline silicon; more preferably polycrystalline silicon. Polycrystalline silicon is cheaper than single crystalline silicon, but because the polycrystalline has more grain boundary, a defect usually occurs in the grain boundary that hinders electron transduction. Such phenomenon makes the solid surface uneven and quantification difficult. Furthermore, ions may penetrate into the grain boundary of the polycrystalline and cause detection failure in solution. In addition, polycrystalline silicon is not stable in air. The abovementioned drawbacks, however, would not interfere with the function of the method according to the invention.

The manner of attaching the recognizing single-stranded oligonucleotide molecule and the solid surface depends on the material of the solid surface and the type of recognizing single-stranded oligonucleotide molecule. In one embodiment of the invention, the recognizing single-stranded oligonucleotide molecule links to the solid surface through a covalent bond. Examples of the covalent bond include but are not limited to the following methods, depending on the solid surface chemistry and the modification of the oligonucleotide. In one embodiment of the invention, when silicon oxide is used as the solid surface, the solid surface is modified by using (3-Aminopropyl)triethoxysilane (APTES). The silicon atom in the molecule of APTES performs a covalent bond with the oxygen atom of the hydroxyl group and it converts the surface's silanol groups (SiOH) to amines; then the 5'-amino group of recognizing single-stranded oligonucleotide molecule is covalently bonded with the solid surface amines group by glutaraldehyde (Roey Elnathan, Moria Kwiat, Alexander Pevzner, Yoni Engel, Larisa Burstein Artium Khatchtourints, Amir Lichtenstein, Raisa Kantaev, and Fernando Patolsky, Biorecognition Layer Engineering: Overcoming Screening Limitations of Nanowire-Based FET Devices, Nano letters, 2012, 12, 5245-5254). In another embodiment of the invention, the solid surface is modified into self-assembling monolayer molecules with different functional groups for covalently linking to different functional groups of the recognizing single-stranded oligonucleotide molecule by various chemical reactions (Srivatsa Venkatasubbarao, Microarrays - status and prospects, TRENDS in Biotechnology Vol. 22 No. 12 December 2004; Ki Su Kim, Hyun-Seung Lee, Jeong-A Yang, Moon-Ho Jo and Sei Kwang Hahn, The fabrication, characterization and application of aptamer-functionalized Si-nanowire FET biosensors, Nanotechnology 20 (2009)).

In another preferred embodiment of the invention, the recognizing single-stranded oligonucleotide molecule is spaced apart from the solid surface by a distance. Since the electrical change detecting element is applied for detecting the electrical change due to the binding of the electrically charged methylation detecting molecule and the target oligonucleotide molecule, the recognizing single-stranded oligonucleotide molecule is not necessary to directly bind to the solid surface, provided that the distance between the recognizing single-stranded oligonucleotide molecule and the solid surface is short enough to allow the electrical change detecting element to detect the electrical change. Preferably, the distance between the solid surface and the recognizing single-stranded oligonucleotide molecule is about 0 to about 10 nm; more preferably about 0 to about 5 nm.

Preferably, the solid surface is coupled with the electrical change detecting element for detecting the electrical change.

According to the invention, a free form of the electrically charged methylation detecting molecule is preferably removed before detecting the electrical change, thereby avoiding noise generated by the electrical charges carried by the free form of the electrically charged methylation detecting molecule. The manner of removal includes but is not limited to washing.

In one preferred embodiment of the invention, the method is for detecting the position of the methylation of the target oligonucleotide molecule. By attaching the recognizing single-stranded oligonucleotide molecule on the solid surface, the electrical changes occurring in different positions of the duplex are able to be distinguished from each other. For example, when the electrically charged methylation detecting molecule binds to the methylated cytosine nucleotide positioned near the solid surface, a stronger electrical signal is detected by the electrical change detecting element coupled with the solid surface, because the electrical charges introduced by the electrically charged methylation detecting molecule are positioned near the electrical change detecting element. On the other hand, when the electrically charged methylation detecting molecule binds to the methylated cytosine nucleotide positioned away from the solid surface, a weaker electrical signal is detected by the electrical change detecting element coupled with the solid surface, because the electrical charges introduced by the electrically charged methylation detecting molecule are positioned away from the electrical change detecting element.

In one preferred embodiment of the invention, the method is for detecting the number or percentage of the methylation of the target oligonucleotide molecule. By attaching the recognizing single-stranded oligonucleotide molecule on the solid surface, the electrical changes occurring due to different numbers of methylated cytosine nucleotide of the duplex are able to be distinguished from each other. For example, when more electrically charged methylation detecting molecules bind to the target oligonucleotide molecule with more methylated cytosine nucleotides, a stronger electrical signal is detected by the electrical change detecting element coupled with the solid surface, because more electrical charges are introduced by the electrically charged methylation detecting molecule. On the other hand, when less electrically charged methylation detecting molecules bind to the target oligonucleotide molecule with less methylated cytosine nucleotides, a weaker electrical signal is detected by the electrical change detecting element coupled with the solid surface, because less electrical charges are introduced by the electrically charged methylation detecting molecule.

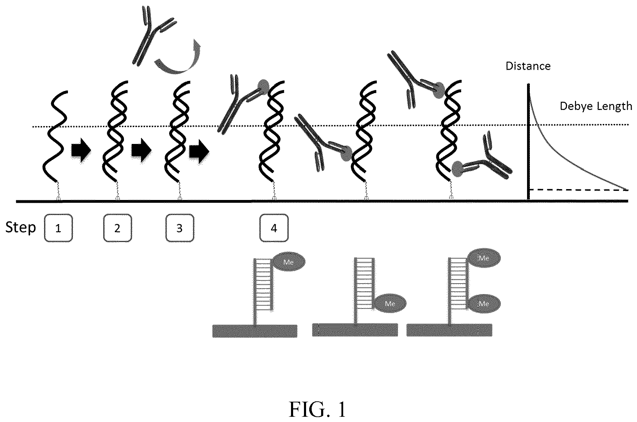

Referring to FIG. 1, a preferred embodiment of the invention is illustrated. In step 1, a recognizing single-stranded oligonucleotide molecule is attached to a solid surface. In step 2, a target oligonucleotide molecule is captured by the recognizing single-stranded oligonucleotide molecule to form a duplex according to base complementarity. In step 3, an electrically charged methylation detecting molecule is applied for contacting the duplex. In step 4, because the target oligonucleotide molecule comprises at least one methylated cytosine nucleotide, the electrically charged methylation detecting molecule binds to the least one methylated cytosine nucleotide, and an electrical change occurs due to the binding of an electrically charged methylation detecting molecule and the target oligonucleotide molecule.

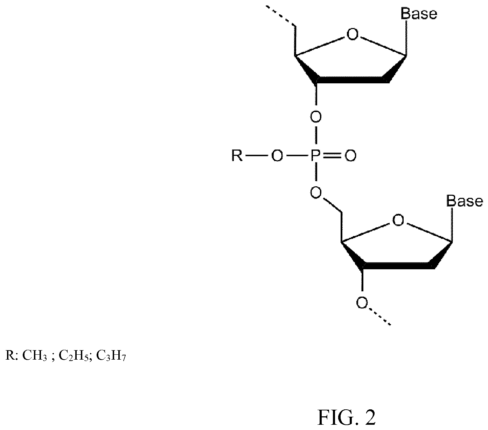

In one preferred embodiment of the invention, the recognizing single-stranded oligonucleotide molecule is a partially neutral single-stranded oligonucleotide comprising at least one electrically neutral nucleotide and at least one negatively charged nucleotide. The manner of rendering a nucleotide electrically neutral is not limited. In one embodiment of the invention, the electrically neutral nucleotide comprises a phosphate group substituted by an alkyl group. Preferably, the alkyl group is a $C_1$-$C_6$ alkyl group; more preferably, the alkyl group is a $C_1$-$C_3$ alkyl group. Examples of the $C_1$-$C_3$ alkyl group include but are not limited to methyl, ethyl and propyl. FIG. 2 shows one preferred embodiment of the electrically neutral nucleotide according to the invention. The negatively-charged oxygen atom in the phosphate group is changed to a neutral atom without charge. The way to substitute the phosphate group with the alkyl group can be applied according to common chemical reactions.

The negatively charged nucleotide according to the invention comprises a phosphate group with at least one negative charge. The unmodified nucleotide is preferably a naturally occurring nucleotide without modification or substitution. In one preferred embodiment of the invention, the negatively charged nucleotide comprises an unsubstituted phosphate group.

The partially neutral single-stranded oligonucleotide according to the invention is partially rendered electrically neutral. The sequence or length is not limited, and the sequence or length of the partially neutral single-stranded oligonucleotide can be designed according to a target oligonucleotide molecule based on the disclosure of the invention.

The numbers of electrically neutral nucleotides and negatively charged nucleotides depend on the sequence of the partially neutral single-stranded oligonucleotide and the condition under the duplex formation. The positions of the electrically neutral nucleotides and negatively charged nucleotides also depend on the sequence of the partially neutral single-stranded oligonucleotides and the condition under the duplex formation. The numbers and positions of the electrically neutral nucleotides and negatively charged nucleotides can be designed according to the available information based on the disclosure of the invention. For example, the number and position of the electrically neutral nucleotides can be designed by molecular modeling calculation based on double stranded (ds) structural energy, and the melting temperature (Tm) of dsDNA/DNA or dsDNA/RNA can then be determined by reference to the structural energy.

In one preferred embodiment of the invention, the partially neutral single-stranded oligonucleotide comprises a plurality of the electrically neutral nucleotides, and at least one negatively charged nucleotide is positioned between two of the electrically neutral nucleotides; more preferably, at least two negatively charged nucleotides are positioned between two of the electrically neutral nucleotides.

In one preferred embodiment of the invention, when the partially neutral single-stranded oligonucleotide according to the invention is applied, at least one electrically neutral nucleotide is positioned near the methylated cytosine nucleotide; more preferably, 2, 3, 4, or 5 electrically neutral nucleotides are positioned near the methylated cytosine nucleotide. In another aspect, at least one electrically neutral nucleotide is positioned at the downstream or upstream site of the methylated cytosine nucleotide; preferably, at least one electrically neutral nucleotide is positioned at the downstream and upstream sites of the methylated cytosine nucleotide.

By introducing the electrically neutral nucleotide, the melting temperature difference between perfect match double-stranded oligonucleotides and mismatched double-stranded oligonucleotides of the partially neutral single-stranded oligonucleotide according to the invention is higher compared with that of a conventional DNA probe. Without being restricted by theory, it is surmised that the electrostatic repulsion force between two strands is lowered by introducing the neutral oligonucleotide, and the melting temperature is raised thereby. By controlling the number and position of electrically neutral nucleotides, the melting temperature difference is adjusted to a desired point, providing a better working temperature or temperature range to differentiate the perfect and mismatched oligonucleotides, thereby improving capture specificity. Such design benefits consistency of the melting temperature of different partially neutral single-stranded oligonucleotides integrated in one chip or array. The number of reactions to be detected can be raised dramatically with high specificity and more detection units can be incorporated into a single detection system. The design provides better microarray operation conditions.

In one preferred embodiment of the invention, the partially neutral single-stranded oligonucleotide comprises a first portion attached to the solid surface; the length of the first portion is about 50% of the total length of the partially neutral single-stranded oligonucleotide; and the first portion comprises at least one electrically neutral nucleotide and at least one negatively charged nucleotide; more preferably, the length of the first portion is about 40% of the total length of the partially neutral single-stranded oligonucleotide; still more preferably, the length of the first portion is about 30% of the total length of the partially neutral single-stranded oligonucleotide.

In one preferred embodiment of the invention, the partially single-stranded nucleotide further comprises a second portion adjacent to the first portion. The second portion is located in the distal end to the solid surface. The second portion comprises at least one electrically neutral nucleotide and at least one negatively charged nucleotide. The description of the electrically neutral nucleotide and the negatively charged nucleotide is the same as that of the first portion and is not repeated herein.

In one preferred embodiment of the invention, a signal amplifier is further provided for enhancing the detection of the electrical change. The signal amplifier according to the invention preferably refers to a component that enhances the detection of the voltage change of the solid surface. For example, the signal amplifier can be nanogold particles attached to one end of the recognizing single-stranded oligonucleotide molecule.

In one preferred embodiment of the invention, the method is performed in a buffer with an ionic strength lower than about 50 mM; more preferably lower than about 40 mM, 30 mM, 20 mM or 10 mM. Without being restricted by theory, it is surmised that by applying the partially neutral single-stranded oligonucleotide, the duplex formed between the partially neutral single-stranded oligonucleotide with the target oligonucleotide molecule can happen without the need to suppress the electrostatic repulsive forces between the partially charged semi-neutral single-stranded oligonucleotide and its target. The hybridization is then driven by the base pairing and the stacking force of each strand. Consequently, the duplex can be formed at a lower salt condition. With FET, the lower ion strength increases the detection length (the debye length) and, in turn, enhances the detection sensitivity.

In one embodiment of the invention, the improved hybridization specificity for forming the duplex can be seen mainly in two aspects of FET detection compared to a conventional detection. First, the melting temperature difference is higher. Second, the buffer has a lower salt condition, and the FET detection length (the debye length) is increased.

Both of these differences result in improvement of detection sensitivity.

In a preferred embodiment of the invention, several recognizing single-stranded oligonucleotide molecules are contained in one system to carry out several detections in one manipulation. For example, a plurality of recognizing single-stranded oligonucleotide molecules may be incorporated in a detection system. Preferably, the detection system is a microarray or a chip.

In one preferred embodiment of the invention, the method comprises steps of:

(a) providing

the single strand of the target oligonucleotide molecule;

the recognizing single-stranded oligonucleotide molecule;

a reference single-stranded oligonucleotide molecule having the target sequence without methylated cytosine nucleotide; and

the electrically charged methylation detecting molecule;

(b) capturing the single strand of the target oligonucleotide molecule with the recognizing single-stranded oligonucleotide molecule to form a target duplex, and capturing the reference single-stranded oligonucleotide molecule with the recognizing single-stranded oligonucleotide molecule to form a reference duplex, respectively;

(c) contacting the electrically charged methylation detecting molecule with the target duplex to form a target complex, and contacting the electrically charged methylation detecting molecule with the reference duplex, respectively; and removing a free form of the electrically charged methylation detecting molecule; and

(d) monitoring an electrical change between the target complex and the reference duplex.

As used herein, the term "a reference single-stranded oligonucleotide molecule" refers to an oligonucleotide molecule that has the target sequence without methylated cytosine nucleotide, providing a background with no methylation. Preferably, the reference single-stranded oligonucleotide molecule has the same structure as the target oligonucleotide molecule except the methylated cytosine nucleotides. In another aspect, the reference single-stranded oligonucleotide molecule and the target oligonucleotide molecule are both captured by the recognizing single-stranded oligonucleotide molecule, and the manipulations relating to the reference single-stranded oligonucleotide molecule and the target oligonucleotide molecule in steps (b) to (d) are performed simultaneously.

Preferably, the conditions for forming the target duplex and the reference duplex are the same. In another aspect, the conditions for forming the target complex and the reference complex are the same.

The electrical change between the target complex and the reference duplex represents the electrical change occurring due to the binding of the electrically charged methylation detecting molecule and the target oligonucleotide molecule.

The present invention is also to provide a kit for detecting methylation of a target oligonucleotide molecule comprising:

an electrically charged methylation detecting molecule having affinity to a methylated cytosine nucleotide; and

an electrical change detecting element for detecting an electrical change.

Preferably, the kit according to the invention further comprises the recognizing single-stranded oligonucleotide molecule as mentioned above.

Preferably, the kit according to the invention further comprises the signal amplifier as mentioned above.

Preferably, the kit according to the invention further comprises the reference single-stranded oligonucleotide molecule having the target sequence without methylated cytosine nucleotide as mentioned above.

The following examples are provided to aid those skilled in the art in practicing the present invention.

**EXAMPLES**

*Synthesis of partially neutral single-stranded oligonucleotide as recognizing single-stranded oligonucleotide molecule:*

Deoxy cytidine (n-ac) p-methoxy phosphoramidite, thymidine p-methoxy phosphoramidite, deoxy guanosine (n-ibu) p-methoxy phosphoramidite, and deoxy adenosine (n-bz) p-methoxy phosphoramidite (all purchased from Chem-Genes Corporation, USA) were used to synthesize an oligonucleotide according to a given sequence based on solid-phase phosphotriester synthesis or by Applied Biosystems 3900 High Throughput DNA Synthesizer (provided by Genomics® Biosci & Tech or Mission Biotech).

The synthesized oligonucleotide was reacted with weak alkaline in toluene at room temperature for 24 hours, and the sample was subjected to ion-exchange chromatography to adjust the pH value to 7. After the sample was concentrated and dried, the partially neutral single-stranded oligonucleotide was obtained.

*Recognizing single-stranded oligonucleotide molecule attachment:*

Recognizing single-stranded oligonucleotide molecule attachment was performed by functionalization of the Si-nanowire (SiNW) surface layer (SiO$_2$) First, (3-Aminopropyl)triethoxysilane (APTES) was used to modify the surface. The silicon atom in the molecule of APTES performed a covalent bond with the oxygen of the hydroxyl group and converted the surface's silanol groups (SiOH) to amines. Samples were immersed in 2% APTES (99% EtOH) for 30 minutes and then heated to 120°C for 10 min. After this step was completed, amino groups (NH$_2$) were the terminal units from the surface.

Next, glutaraldehyde was used as a grafting agent for DNA attachment. Glutaraldehyde binding was achieved through its aldehyde group (COH) to ensure a covalent bond with the amino group of APTES. For this step, samples were immersed in 12.5% glutaraldehyde (10mM sodium phosphate buffer) in liquid for 1 hour at room temperature.

The recognizing single-stranded oligonucleotide molecule was mixed with a bis-tris propane buffer at the concentration of 1 $\mu$M, and the modified SiNW surface was further immersed in the solution for 12 hours.

*Capturing target oligonucleotide molecule*

The sequences of the recognizing single-stranded oligonucleotide molecule (probe) and the target oligonucleotide molecule (target) are listed in Table 1.

Table 1:

| Sequence name | DNA sequence (5'→3') | SEQ ID No. |
|---|---|---|
| SEPT9 DNA probe 1 | NH2-GCGCGCTGGCTGGGGCGCCGCGCCCGCGCT | 1 |
| SEPT9 DNA target 1 | AGCGCGGGCGCGGCGCCCCAGCCAGCGCGC | 2 |
| SEPT9 methyl DNA target 1 (C5) | AGCGCmeGGGCGCGGCGCCCCAGCCAGCGCGC | 3 |
| SEPT9 methyl DNA target 2 (C14) | AGCGCGGGCGCGGCmeGCCCCAGCCAGCGCGC | 4 |
| SEPT9 methyl DNA target 3 (C26) | AGCGCGGGCGCGGCGCCCCAGCCAGCmeGCGC | 5 |
| SEPT9 DNA probe 2 | NH2-CGAAATGATCCCATCCAGCTGCGCGTTGACCGC | 6 |
| SEPT9 DNA target 2 | GCGGTCAACGCGCAGCTGGATGGGATCATTTCG | 7 |
| SEPT9 methyl DNA target 4 (C2) | GCmeGGTCAACGCGCAGCTGGATGGGATCATTTCG | 8 |
| SEPT9 methyl DNA target 5 (C2,16) | GCmeGGTCAACGCGCAGCmeTGGATGGGATCATTTCG | 9 |
| SEPT9 methyl DNA target 6 (C2,16,32) | GCmeGGTCAACGCGCAGCmeTGGATGGGATCATTTCmeG | 10 |

(continued)

| Sequence name | DNA sequence (5'→3') | SEQ ID No. |
|---|---|---|
| SEPT9 methyl DNA target 7 (C39) | GCGGTCAACGCGCAGCTGGATGGGATCATTTCGGACT TCmeGAAGGTGGG | 11 |
| SEPT9 N-DNA probe | NH2-CnGAAnATGnATCnCCAnTCCnAGCTGCGCGTTGA CCGC | 12 |
| $C_{me}$: methylated cytosine nucleotide | | |

Example 1: Detecting the position of the methylation of the target oligonucleotide molecule

The target oligonucleotide molecules were SEPT9 methyl DNA target 1 (C5), SEPT9 methyl DNA target 1 (C14), and SEPT9 methyl DNA target 1 (C26), having a methylated cytosine nucleotide at positions 5, 14, and 26, respectively.

The target oligonucleotide molecule was mixed with a bis-tris propane buffer at the concentration of 100 pM, and captured by SEPT9 DNA probe 1 attached to the SiNW for 30 minutes to form a duplex.

Anti-5mC antibody (0.25 $\mu$g/mL) as an electrically charged methylation detecting molecule was introduced to the duplex for reacting for 30 minutes.

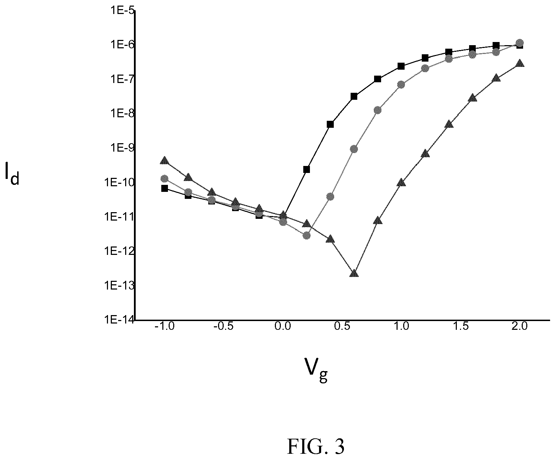

The results are shown in FIGs. 3 to 7.

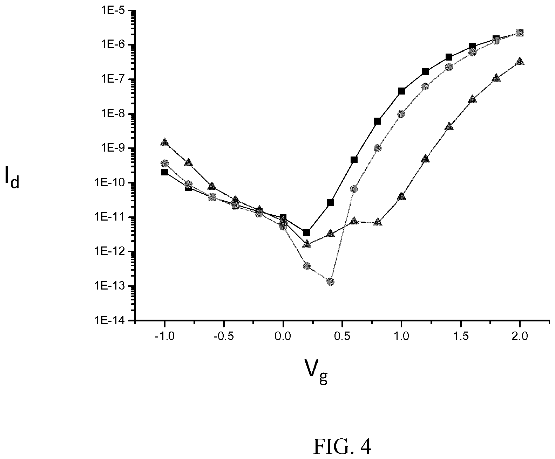

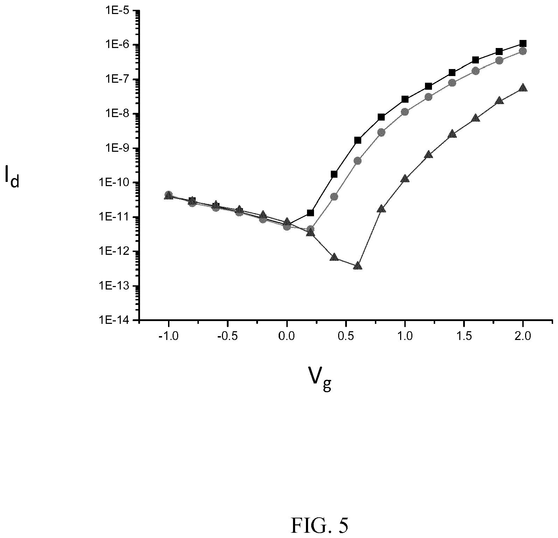

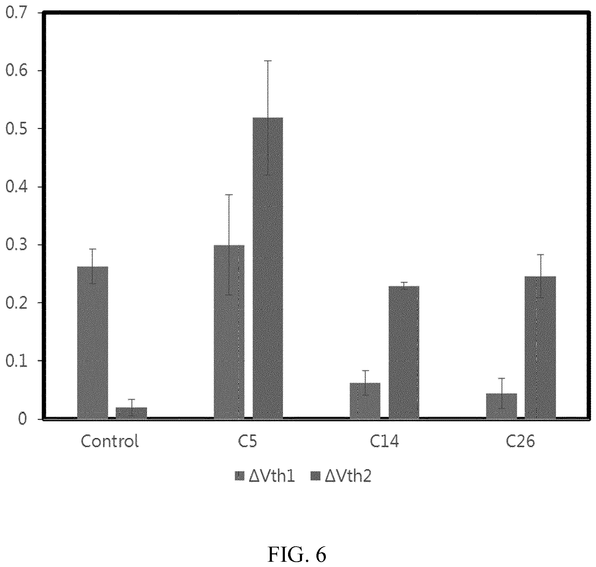

FIG. 3 shows the electrical signal of pSNWFET under analyte processing. "■" line showed the $I_d$-$V_g$ curve of pSNWFET processed with 10 mM, pH=7 bis-tris propane buffer. After the pSNWFET processed with the complementary DNA, the DNA compromised buffer was replaced with the same bis-tris propane buffer. Subsequently, electrical signal detection was processed and resulted as the "●" line. Finally, buffer comprising the anti-methylcytosine antibody was injected and incubated on nanowire. Replacement of bis-tris propane buffer was followed by the incubation, and the electrical signal is shown as the "▲" " line. Similar detections for different methylcytosine sites were performed and the results are shown in FIG. 4 and FIG. 5. The average $\Delta V_{th}$ induced by target DNA and anti-methylcytosine antibody is shown in FIG. 6. The following equation is used to normalize the electrical signal induced by antibody:

$$\frac{\Delta V_{th} \text{ induced by antibody}}{\Delta V_{th} \text{ induced by target DNA}}$$

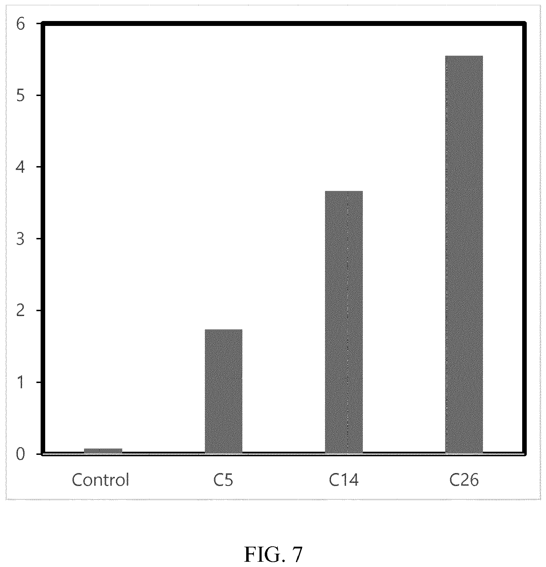

FIG. 7 shows the result of normalization - ratio of antibody to target DNA, which significantly demonstrates that the method according to the invention is for detecting the positions of the methylation of the target oligonucleotide molecule. Example 2: Detecting the number of the methylated cytosine nucleotides in the target oligonucleotide molecule

The target oligonucleotide molecules were SEPT9 methyl DNA target 4 (C2), SEPT9 methyl DNA target 5 (C2+C16), and SEPT9 methyl DNA target 6 (C2+C16+C32), having one, two or three methylated cytosine nucleotides, respectively.

The target oligonucleotide molecule was mixed with a bis-tris propane buffer at the concentration of 100 pM, and captured by SEPT9 DNA probe 1 attached to the SiNW for 30 minutes to form a duplex.

Anti-5mC antibody (0.25 $\mu$g/mL) as an electrically charged methylation detecting molecule was introduced to the duplex for reacting for 30 minutes.

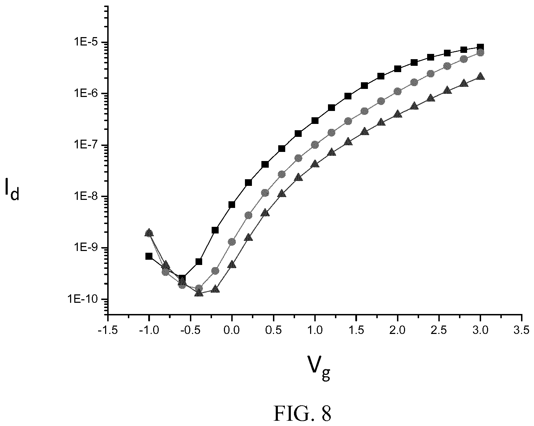

The results are shown in FIGs. 8 to 10.

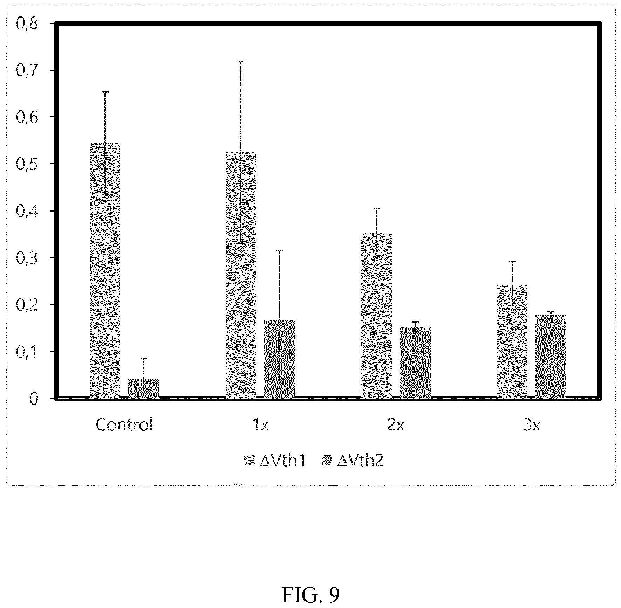

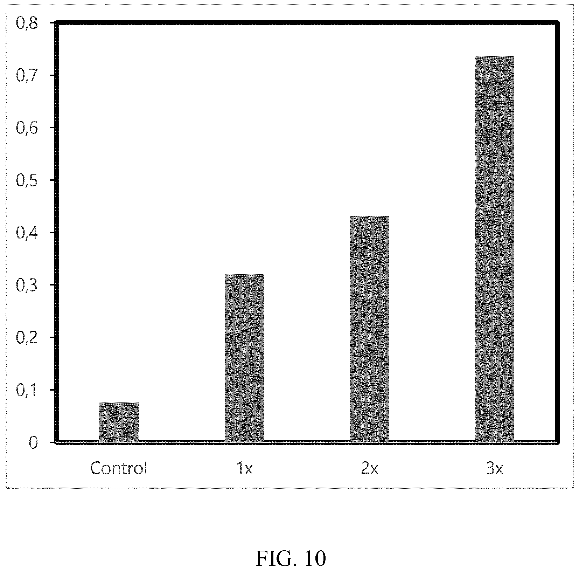

The target oligonucleotide molecules were synthesized with different numbers of methylcytosine, which provides different numbers of antibody binding sites. FIG. 8 shows the detection result of SEPT9 methyl DNA target 6. As the validation of distance effect, $I_d$-$V_g$ curve was detected in the condition of bis-tris propane buffer, buffer containing DNA target and anti-methylcytosine antibody. FIG. 9 demonstrates the average of 3 times threshold voltage shift induced by the target DNA and antibody, respectively. FIG. 10 shows the normalization of threshold voltage shifts from FIG. 9, and it obviously indicates that the numbers of methylcytosine have positive correlation with the voltage shift.

Example 3: Detecting the methylated cytosine nucleotides on a single strand tail in the target oligonucleotide molecule

The target oligonucleotide molecule was SEPT9 methyl DNA target 7 (C39).

The target oligonucleotide molecule was mixed with a bis-tris propane buffer at the concentration of 100 pM, and captured by SEPT9 DNA probe 2 attached to the SiNW for 30 minutes to form a duplex.

Anti-5mC antibody (1 ng/mL) as an electrically charged methylation detecting molecule was introduced to the duplex for reacting for 30 minutes.

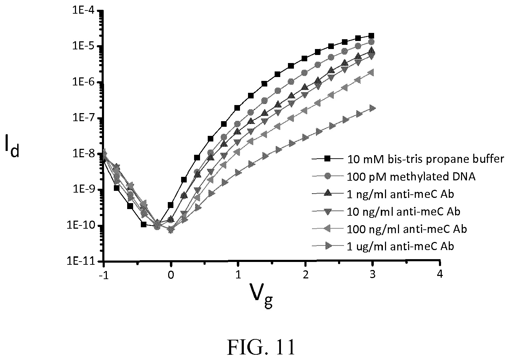

The results are shown in FIG. 11. The figure demonstrates that the methylated cytosine positioned on a single strand tail of the duplex formed by the SEPT9 DNA probe 2 with SEPT9 methyl DNA target 7 increases the detection limit to 1 ng/ml anti-methylcytosine antibody.

Example 4: Detecting the methylated cytosine nucleotides in the target oligonucleotide molecule with N-DNA

The target oligonucleotide molecule was SEPT9 methyl DNA target 6 (C2,16,32).

The target oligonucleotide molecule was mixed with a bis-tris propane buffer at the concentration of 100 pM, and captured by SEPT9 N-DNA probe attached to the SiNW for 30 minutes to form a duplex.

Anti-5mC antibody (0.1 1 $\mu$g/mL) as an electrically charged methylation detecting molecule was introduced to the duplex for reacting for 30 minutes.

The results are shown in FIG. 11. The figure demonstrates that the methylated cytosine positioned on a single strand tail of the duplex formed by the SEPT9 DNA probe 2 with SEPT9 methyl DNA target 7 increases the detection limit to 1 ng/ml anti-methylcytosine antibody.

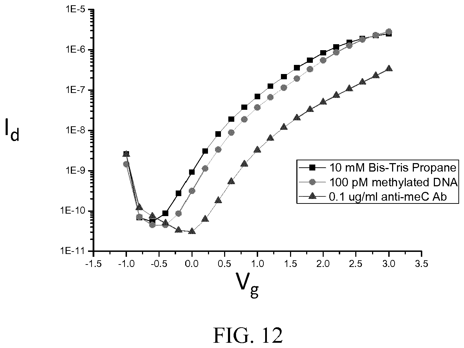

FIG. 12 shows $I_d$-$V_g$ curve of partially neutral single-stranded oligonucleotide (N-DNA) probe binding with SEPT9 methyl DNA target 6 (C2, C16, C32) and antibody. Compared to FIG. 8, it shows that N-DNA has the improved effect in sensitivity.

While the present invention has been described in conjunction with the specific embodiments set forth above, many alternatives thereto and modifications and variations thereof will be apparent to those of ordinary skill in the art. All such alternatives, modifications and variations are regarded as falling within the scope of the present invention.

**Claims**

1. A method for detecting methylation of a target oligonucleotide molecule, comprising obtaining an electrical change occurring due to the binding of an electrically charged methylation detecting molecule and the target oligonucleotide molecule; wherein the electrically charged methylation detecting molecule has affinity to a methylated cytosine nucleotide, wherein the target oligonucleotide molecule has a target sequence with at least one methylated cytosine nucleotide, and the method comprises:

   capturing a single strand of the target oligonucleotide molecule by a recognizing single-stranded oligonucleotide molecule to form a duplex according to base complementarity;
   providing the electrically charged methylation detecting molecule; and
   binding the duplex with the electrically charged methylation detecting molecule and detecting the electrical change due to the binding,

   wherein the recognizing single-stranded oligonucleotide molecule is a partially neutral single-stranded oligonucleotide comprising at least one electrically neutral nucleotide and at least one negatively charged nucleotide.

2. The method according to claim 1, wherein the electrically charged methylation detecting molecule is selected from the group consisting of an anti-methylcytosine antibody, a methyl binding domain protein and a restriction enzyme.

3. The method according to claim 1, wherein the electrical change is a threshold voltage shift change.

4. The method according to claim 1, wherein the duplex formed by the single strand of the target oligonucleotide molecule and the recognizing single-stranded oligonucleotide molecule comprises a bulge and the methylated cytosine nucleotide is disposed in the bulge.

5. The method according to claim 1, wherein the recognizing single-stranded oligonucleotide molecule is attached to a solid surface or the recognizing single-stranded oligonucleotide molecule is spaced apart from the solid surface by a distance.

6. The method according to claim 5, wherein the solid surface is a transistor surface of a field-effect transistor (FET) or a metal surface of a surface plasmon resonance (SPR).

7. The method according to claim 5, wherein the material of the solid surface is polycrystalline silicon or single crystalline silicon.

8. The method according to claim 5, wherein the solid surface is coupled with an electrical change detecting element for detecting the electrical change.

9. The method according to claim 8, wherein the electrical change detecting element is a field-effect transistor or a surface plasmon resonance.

10. The method according to claim 1, wherein the electrically neutral nucleotide comprises a phosphate group substituted by a $C_1$-$C_6$ alkyl group.

11. The method according to claim 1, wherein the negatively charged nucleotide comprises an unsubstituted phosphate group.

12. The method according to claim 1, wherein the recognizing single-stranded oligonucleotide molecule is attached to a solid surface, and the partially neutral single-stranded oligonucleotide comprises a first portion attached to the solid surface; the length of the first portion is about 50% of the total length of the partially neutral single-stranded oligonucleotide; and the first portion comprises at least one electrically neutral nucleotide and at least one negatively charged nucleotide.

13. The method according to claim 1, comprising steps of:

    (a) providing

        the single strand of the target oligonucleotide molecule;
        the recognizing single-stranded oligonucleotide molecule;
        a reference single-stranded oligonucleotide molecule having the target sequence without methylated cytosine nucleotide; and
        the electrically charged methylation detecting molecule;

    (b) capturing the single strand of the target oligonucleotide molecule with the recognizing single-stranded oligonucleotide molecule to form a target duplex, and capturing the reference single-stranded oligonucleotide molecule with the recognizing single-stranded oligonucleotide molecule to form a reference duplex, respectively;
    (c) contacting the electrically charged methylation detecting molecule with the target duplex to form a target complex, and contacting the electrically charged methylation detecting molecule with the reference duplex, respectively; and removing a free form of the electrically charged méthylation detecting molecule; and
    (d) monitoring an electrical change between the target complex and the reference duplex.

14. A kit for detecting methylation of a target oligonucleotide molecule comprising:

    an electrically charged methylation detecting molecule having affinity to a methylated cytosine nucleotide;
    an electrical change detecting element for detecting an electrical change; and
    a recognizing single-stranded oligonucleotide molecule which is able to capture a single strand of the target oligonucleotide molecule to form a duplex according to base complementarity, wherein the recognizing single-stranded DNA molecule is a partially neutral single-stranded oligonucleotide comprising at least one electrically neutral nucleotide and at least one negatively charged nucleotide.

15. The kit according to claim 14, wherein the electrically charged methylation detecting molecule is selected from the group consisting of an anti-methylcytosine antibody, a methyl binding domain protein and a restriction enzyme.

16. The kit according to claim 14, wherein the recognizing single-stranded oligonucleotide molecule is able to form a duplex comprising a bulge with the single strand of the target oligonucleotide molecule, and the methylated cytosine nucleotide is disposed in the bulge.

17. The kit according to claim 16, wherein the recognizing single-stranded oligonucleotide molecule is attached to a solid surface or the recognizing single-stranded oligonucleotide molecule is spaced apart from the solid surface by a distance.

18. The kit according to claim 17, wherein the solid surface is a transistor surface of a field-effect transistor or a metal surface of a surface plasmon resonance.

19. The kit according to claim 17, wherein the material of the solid surface is polycrystalline silicon or single crystalline silicon.

20. The kit according to claim 14, wherein the electrical change detecting element is a field-effect transistor or a surface plasmon resonance.

21. The kit according to claim 14, wherein the electrically neutral nucleotide comprises a phosphate group substituted by a $C_1$-$C_6$ alkyl group.

22. The kit according to claim 14, wherein the negatively charged nucleotide comprises an unsubstituted phosphate group.

23. The kit according to claim 14, wherein the recognizing single-stranded oligonucleotide molecule is attached to a solid surface, and the partially neutral single-stranded oligonucleotide comprises a first portion attached to the solid surface; the length of the first portion is about 50% of the total length of the partially neutral single-stranded oligonucleotide; and the first portion comprises at least one electrically neutral nucleotide and at least one negatively charged nucleotide.

24. The kit according to claim 14 further comprising a reference single-stranded oligonucleotide molecule having the target sequence without methylated cytosine nucleotide.

**Patentansprüche**

1. Verfahren zum Nachweisen von Methylierung eines Ziel-Oligonukleotidmoleküls, umfassend Erhalten einer elektrischen Änderung, die aufgrund des Bindens von einem elektrisch geladenen Methylierungsnachweismolekül und dem Ziel-Oligonukleotidmolekül auftritt; wobei das elektrisch geladene Methylierungsnachweismolekül Affinität zu einem methylierten Cytosinnukleotid aufweist, wobei das Ziel-Oligonukleotidmolekül eine Zielsequenz mit mindestens einem methylierten Cytosinnukleotid aufweist, und das Verfahren umfasst:

   Einfangen eines Einzelstranges des Ziel-Oligonukleotidmoleküls durch ein einzelsträngiges Erkennungs-Oligonukleotidmolekül, wobei ein Duplex gemäß Basenkomplementarität gebildet wird;
   Bereitstellen des elektrisch geladenen Methylierungsnachweismoleküls; und
   Binden des Duplexs an dem elektrisch geladenen Methylierungsnachweismolekül und Nachweisen der elektrischen Änderung aufgrund der Bindung,
   wobei das einzelsträngige Erkennungs-Oligonukleotidmolekül ein partiell neutrales einzelsträngiges Oligonukleotid ist, umfassend mindestens ein elektrisch neutrales Nukleotid und mindestens ein negativ geladenes Nukleotid.

2. Verfahren gemäß Anspruch 1, wobei das elektrisch geladene Methylierungsnachweismolekül aus der Gruppe ausgewählt ist, welche aus einem Anti-Methylcytosin-Antikörper, einem Methyl-Binde-Domäne-Protein und einem Restriktionsenzym besteht.

3. Verfahren gemäß Anspruch 1, wobei die elektrische Änderung eine Änderung der Schwellspannungsverschiebung ist.

4. Verfahren gemäß Anspruch 1, wobei das Duplex, welches durch den Einzelstrang des Ziel-Oligonukleotidmoleküls und das einzelsträngige Erkennungs-Oligonukleotidmolekül gebildet wird, eine einseitige Ausbuchtung (bulge) umfasst und sich das methylierte Cytosinnukleotid in der Ausbuchtung befindet.

5. Verfahren gemäß Anspruch 1, wobei das einzelsträngige Erkennungs-Oligonukleotidmolekül an einer festen Oberfläche angebracht ist oder das einzelsträngige Erkennungs-Oligonukleotidmolekül von der festen Oberfläche durch einen Abstand räumlich getrennt ist.

6. Verfahren gemäß Anspruch 5, wobei die feste Oberfläche eine Transistoroberfläche eines Feldeffekt-Transistors (FET) oder eine Metalloberfläche einer Oberflächenplasmonenresonanz (SPR) ist.

7. Verfahren gemäß Anspruch 5, wobei das Material der festen Oberfläche polykristallines Silicium oder einzelkristallines Silicium ist.

**8.** Verfahren gemäß Anspruch 5, wobei die feste Oberfläche zum Nachweisen der elektrischen Änderung mit einem Element zum Nachweis einer elektrischen Änderunggekoppelt ist.

**9.** Verfahren gemäß Anspruch 8, wobei das Element zum Nachweis einer elektrischen Änderung ein Feldeffekt-Transistor oder eine Oberflächenplasmonenresonanz ist.

**10.** Verfahren gemäß Anspruch 1, wobei das elektrisch neutrale Nukleotid eine Phosphatgruppe, substituiert mit einem $C_1$-$C_6$-Alkylrest, umfasst.

**11.** Verfahren gemäß Anspruch 1, wobei das negativ geladene Nukleotid eine nicht substituierte Phosphatgruppe umfasst.

**12.** Verfahren gemäß Anspruch 1, wobei das einzelsträngige Erkennungs-Oligonukleotidmolekül an einer festen Oberfläche angebracht ist und das partiell neutrale einzelsträngige Oligonukleotid einen ersten Anteil, angebracht an die feste Oberfläche, umfasst; die Länge des ersten Anteils etwa 50 % der Gesamtlänge des partiell neutralen einzelsträngigen Oligonukleotids beträgt; und der erste Anteil mindestens ein elektrisch neutrales Nukleotid und mindestens ein negativ geladenes Nukleotid umfasst.

**13.** Verfahren gemäß Anspruch 1, umfassend Schritte von:

(a) Bereitstellen

des Einzelstranges des Ziel-Oligonukleotidmoleküls;
des einzelsträngigen Erkennungs-Oligonukleotidmoleküls;
eines einzelsträngigen Referenz-Oligonukleotidmoleküls mit der Zielsequenz ohne methyliertes Cytosinnukleotid; und
des elektrisch geladenen Methylierungsnachweismoleküls;

(b) Einfangen des Einzelstranges des Ziel-Oligonukleotidmoleküls mit dem einzelsträngigen Erkennungs-Oligonukleotidmolekül, wobei ein Zielduplex gebildet wird, beziehungsweise Einfangen des einzelsträngigen Referenz-Oligonukleotidmoleküls mit dem einzelsträngigen Erkennungs-Oligonukleotidmolekül, wobei ein Referenzduplex gebildet wird;
(c) Inkontaktbringen des elektrisch geladenen Methylierungsnachweismoleküls mit dem Zielduplex, wobei ein Zielkomplex gebildet wird, beziehungsweise Inkontaktbringen des elektrisch geladenen Methylierungsnachweismoleküls mit dem Referenzduplex; und Entfernen einer freien Form des elektrisch geladenen Methylierungsnachweismoleküls; und
(d) Überwachen einer elektrischen Änderung zwischen dem Zielkomplex und dem Referenzduplex.

**14.** Kit zum Nachweisen von Methylierung eines Ziel-Oligonukleotidmoleküls, umfassend:

ein elektrisch geladenes Methylierungsnachweismolekül mit Affinität zu einem methylierten Cytosinnukleotid;
ein Element zum Nachweis einer elektrischen Änderung zum Nachweisen einer elektrischen Änderung; und
ein einzelsträngiges Erkennungs-Oligonukleotidmolekül, welches zum Einfangen eines Einzelstranges des Ziel-Oligonukleotidmoleküls in der Lage ist, wobei ein Duplex gemäß Basenkomplementarität gebildet wird, wobei das einzelsträngige Erkennungs-DNA-Molekül ein partiell neutrales einzelsträngiges Oligonukleotid, umfassend mindestens ein elektrisch neutrales Nukleotid und mindestens ein negativ geladenes Nukleotid, ist.

**15.** Kit gemäß Anspruch 14, wobei das elektrisch geladene Methylierungsnachweismolekül aus der Gruppe ausgewählt ist, welche aus einem Anti-Methylcytosin-Antikörper, einem Methyl-Binde-Domäne-Protein und einem Restriktionsenzym besteht.

**16.** Kit gemäß Anspruch 14, wobei das einzelsträngige Erkennungs-Oligonukleotidmolekül in der Lage ist, mit dem Einzelstrang des Ziel-Oligonukleotidmoleküls ein Duplex umfassend einseitige Ausbuchtung (bulge) zu bilden und sich das methylierte Cytosinnukleotid in der Ausbuchtung befindet.

**17.** Kit gemäß Anspruch 16, wobei das einzelsträngige Erkennungs-Oligonukleotidmolekül an einer festen Oberfläche angebracht ist oder das einzelsträngige Erkennungs-Oligonukleotidmolekül von der festen Oberfläche durch einen Abstand räumlich getrennt ist.

**18.** Kit gemäß Anspruch 17, wobei die feste Oberfläche eine Transistoroberfläche eines Feldeffekt-Transistors oder eine Metalloberfläche einer Oberflächenplasmonenresonanz ist.

**19.** Kit gemäß Anspruch 17, wobei das Material der festen Oberfläche polykristallines Silicium oder einzelkristallines Silicium ist.

**20.** Kit gemäß Anspruch 14, wobei das Element zum Nachweis einer elektrischenÄnderung ein Feldeffekt-Transistor oder eine Oberflächenplasmonenresonanz ist.

**21.** Kit gemäß Anspruch 14, wobei das elektrisch neutrale Nukleotid eine Phosphatgruppe, substituiert mit einem $C_1$-$C_6$-Alkylrest, umfasst.

**22.** Kit gemäß Anspruch 14, wobei das negativ geladene Nukleotid eine nicht substituierte Phosphatgruppe umfasst.

**23.** Kit gemäß Anspruch 14, wobei das einzelsträngige Erkennungs-Oligonukleotidmolekül an einer festen Oberfläche angebracht ist und das partiell neutrale einzelsträngige Oligonukleotid einen ersten Anteil, angebracht an die feste Oberfläche, umfasst; die Länge des ersten Anteils etwa 50 % der Gesamtlänge des partiell neutralen einzelsträngigen Oligonukleotids beträgt; und der erste Anteil mindestens ein elektrisch neutrales Nukleotid und mindestens ein negativ geladenes Nukleotid umfasst.

**24.** Kit gemäß Anspruch 14, ferner umfassend ein einzelsträngiges Referenz-Oligonukleotidmolekül mit der Zielsequenz ohne methyliertes Cytosinnukleotid.

## Revendications

**1.** Procédé de détection de méthylation d'une molécule d'oligonucléotide cible, comprenant une obtention d'une modification électrique se produisant du fait de la liaison d'une molécule de détection de méthylation chargée électriquement et de la molécule d'oligonucléotide cible ; dans lequel la molécule de détection de méthylation chargée électriquement présente de l'affinité pour un nucléotide de cytosine méthylé, dans lequel la molécule d'oligonucléotide cible présente une séquence cible avec au moins un nucléotide de cytosine méthylé, et le procédé comprend :

une capture d'un brin simple de la molécule d'oligonucléotide cible par une molécule d'oligonucléotide simple-brin de reconnaissance pour former un duplex par complémentarité de bases ;
une fourniture de la molécule de détection de méthylation chargée électriquement; et
une liaison du duplex avec la molécule de détection de méthylation chargée électriquement et une détection de la modification électrique due à la liaison,

dans lequel la molécule d'oligonucléotide simple-brin de reconnaissance est un oligonucléotide simple-brin partiellement neutre comprenant au moins un nucléotide électriquement neutre et au moins un nucléotide chargé négativement.

**2.** Procédé selon la revendication 1, dans lequel la molécule de détection de méthylation chargée électriquement est choisie dans le groupe constitué d'un anticorps anti-méthylcytosine, d'une protéine à domaine de liaison méthyl et d'une enzyme de restriction.

**3.** Procédé selon la revendication 1, dans lequel la modification électrique est une modification par décalage de tension de seuil.

**4.** Procédé selon la revendication 1, dans lequel le duplex formée par le brin simple de la molécule d'oligonucléotide cible et la molécule d'oligonucléotide simple-brin de reconnaissance comprend un renflement et le nucléotide de cytosine méthylé est disposé dans le renflement.

**5.** Procédé selon la revendication 1, dans lequel la molécule d'oligonucléotide simple-brin de reconnaissance est attachée à une surface solide ou la molécule d'oligonucléotide simple-brin de reconnaissance est espacée d'une distance de la surface solide.

6. Procédé selon la revendication 5, dans lequel la surface solide est une surface de transistor d'un transistor à effet de champ (FET) ou une surface métallique d'une résonance plasmon de surface (SPR).

7. Procédé selon la revendication 5, dans lequel le matériau de la surface solide est le silicium polycristallin ou le silicium monocristallin.

8. Procédé selon la revendication 5, dans lequel la surface solide est couplée à un élément de détection de modification électrique pour détecter la modification électrique.

9. Procédé selon la revendication 8, dans lequel l'élément de détection de modification électrique est un transistor à effet de champ ou une résonance plasmon de surface.

10. Procédé selon la revendication 1, dans lequel le nucléotide électriquement neutre comprend un groupe phosphate substitué par un groupe alkyle en $C_1$-$C_6$.

11. Procédé selon la revendication 1, dans lequel le nucléotide chargé négativement comprend un groupe phosphate non substitué.

12. Procédé selon la revendication 1, dans lequel la molécule d'oligonucléotide simple-brin de reconnaissance est attachée à une surface solide, et l'oligonucléotide simple-brin partiellement neutre comprend une première partie attachée à la surface solide ; la longueur de la première partie est d'environ 50 % de la longueur totale de l'oligonucléotide simple-brin partiellement neutre ; et la première partie comprend au moins un nucléotide électriquement neutre et au moins un nucléotide chargé négativement.

13. Procédé selon la revendication 1, comprenant les étapes suivantes :

   (a) une fourniture

      du brin simple de la molécule d'oligonucléotide cible ;
      de la molécule d'oligonucléotide simple-brin de reconnaissance ;
      d'une molécule d'oligonucléotide simple-brin de référence ayant la séquence cible sans nucléotide de cytosine méthylé ; et
      de la molécule de détection de méthylation chargée électriquement ;

   (b) une capture du brin simple de la molécule d'oligonucléotide cible avec la molécule d'oligonucléotide simple-brin de reconnaissance pour former un duplex cible, et une capture de la molécule d'oligonucléotide simple-brin de référence avec la molécule d'oligonucléotide simple-brin de reconnaissance pour former un duplex de référence, respectivement ;
   (c) une mise en contact de la molécule de détection de méthylation chargée électriquement avec le duplex cible pour former un complexe cible, et une mise en contact de la molécule de détection de méthylation chargée électriquement avec le duplex de référence, respectivement ; et une élimination d'une forme libre de la molécule de détection de méthylation chargée électriquement ; et
   (d) une surveillance d'une modification électrique entre le complexe cible et le duplex de référence.

14. Kit de détection de méthylation d'une molécule d'oligonucléotide cible comprenant :

   une molécule de détection de méthylation chargée électriquement présentant de l'affinité pour un nucléotide de cytosine méthylé ;
   un élément de détection de modification électrique destiné à détecter une modification électrique ; et
   une molécule d'oligonucléotide simple-brin de reconnaissance adaptée à capturer un brin simple de la molécule d'oligonucléotide cible pour former un duplex par complémentarité de bases, dans lequel la molécule d'ADN simple-brin de reconnaissance est un oligonucléotide simple-brin partiellement neutre comprenant au moins un nucléotide électriquement neutre et au moins un nucléotide chargé négativement.

15. Kit selon la revendication 14, dans lequel la molécule de détection de méthylation chargée électriquement est choisie dans le groupe constitué d'un anticorps anti-méthylcytosine, d'une protéine à domaine de liaison méthyl et d'une

EP 3 118 332 B1

enzyme de restriction.

**16.** Kit selon la revendication 14, dans lequel la molécule d'oligonucléotide simple-brin de reconnaissance est adaptée à former un duplex comprenant un renflement avec le brin simple de la molécule d'oligonucléotide cible, et le nucléotide de cytosine méthylé est disposé dans le renflement.

**17.** Kit selon la revendication 16, dans lequel la molécule d'oligonucléotide simple-brin de reconnaissance est attachée à une surface solide ou la molécule d'oligonucléotide simple-brin de reconnaissance est espacée d'une distance de la surface solide.

**18.** Kit selon la revendication 17, dans lequel la surface solide est une surface de transistor d'un transistor à effet de champ ou une surface métallique de résonance plasmon de surface.

**19.** Kit selon la revendication 17, dans lequel le matériau de la surface solide est le silicium polycristallin ou le silicium monocristallin.

**20.** Kit selon la revendication 14, dans lequel l'élément de détection de modification électrique est un transistor à effet de champ ou une résonance plasmon de surface.

**21.** Kit selon la revendication 14, dans lequel le nucléotide électriquement neutre comprend un groupe phosphate substitué par un groupe alkyle en $C_1$-$C_6$.

**22.** Kit selon la revendication 14, dans lequel le nucléotide chargé négativement comprend un groupe phosphate non-substitué.

**23.** Kit selon la revendication 14, dans lequel la molécule d'oligonucléotide simple-brin de reconnaissance est attachée à une surface solide, et l'oligonucléotide simple-brin partiellement neutre comprend une première partie attachée à la surface solide ; la longueur de la première partie est d'environ 50 % de la longueur totale de l'oligonucléotide simple-brin partiellement neutre ; et la première partie comprend au moins un nucléotide électriquement neutre et au moins un nucléotide chargé négativement.

**24.** Kit selon la revendication 14, comprenant en outre une molécule d'oligonucléotide simple-brin de référence ayant la séquence cible sans nucléotide de cytosine méthylé.

FIG. 1

R: $CH_3$ ; $C_2H_5$; $C_3H_7$

FIG. 2

FIG. 3

FIG. 4

FIG. 5

FIG. 6

FIG. 7

FIG. 8

FIG. 9

FIG. 10

FIG. 11

FIG. 12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **KURITA, RYOJI ; OSAMU NIWA.** DNA methylation analysis triggered by bulge specific immuno-recognition. *Analytical chemistry,* 2012, vol. 84.17, 7533-7538
- **LISTER ; RYAN et al.** Human DNA methylomes at base resolution show widespread epigenomic differences. *Nature,* 2009, vol. 7271 (462), 315-322
- **WEBER, MICHAEL et al.** Chromosome-wide and promoter-specific analyses identify sites of differential DNA methylation in normal and transformed human cells. *Nature genetics,* 2005, vol. 37.8, 853-862
- **CROSS, SALLY H. et al.** Purification of CpG islands using a methylated DNA binding column. *Nature genetics,* 1994, vol. 6.3, 236-244
- **ROEY ELNATHAN ; MORIA KWIAT ; ALEXANDER PEVZNER ; YONI ENGEL ; LARISA BURSTEIN ARTIUM KHATCHTOURINTS ; AMIR LICHTENSTEIN ; RAISA KANTAEV ; FERNANDO PATOLSKY.** Biorecognition Layer Engineering: Overcoming Screening Limitations of Nanowire-Based FET Devices. *Nano letters,* 2012, vol. 12, 5245-5254
- **SRIVATSA VENKATASUBBARAO.** Microarrays - status and prospects. *TRENDS in Biotechnology,* December 2004, vol. 22 (12
- **KI SU KIM ; HYUN-SEUNG LEE ; JEONG-A YANG ; MOON-HO JO ; SEI KWANG HAHN.** The fabrication, characterization and application of aptamer-functionalized Si-nanowire FET biosensors. *Nanotechnology,* 2009, vol. 20

SEQUENCE LISTING

<110> OriZhan Bioscience Limited

<120> DNA METHYLATION DETECTION

<130> None

<160> 12

<170> PatentIn version 3.5

<210> 1
<211> 30
<212> DNA
<213> Artificial Sequence

<220>
<223> DNA probe 1

<400> 1
gcgcgctggc tggggcgccg cgcccgcgct 30

<210> 2
<211> 30
<212> DNA
<213> Artificial Sequence

```
<220>
<223> DNA target 1

<400> 2
agcgcgggcg cggcgcccca gccagcgcgc        30


<210> 3
<211> 30
<212> DNA
<213> Artificial Sequence

<220>
<223> methyl DNA target 1 (C5)

<220>
<221> modified_base
<222> (5)..(5)
<223> m5c

<400> 3
agcgcgggcg cggcgcccca gccagcgcgc        30


<210> 4
<211> 30
<212> DNA
<213> Artificial Sequence

<220>
<223> methyl DNA target 2 (C14)

<220>
<221> modified_base
<222> (14)..(14)
<223> m5c

<400> 4
agcgcgggcg cggcgcccca gccagcgcgc        30


<210> 5
<211> 30
<212> DNA
<213> Artificial Sequence

<220>
<223> methyl DNA target 3 (C26)

<220>
<221> modified_base
<222> (26)..(26)
<223> m5c

<400> 5
agcgcgggcg cggcgcccca gccagcgcgc        30


<210> 6
<211> 33
<212> DNA
<213> Artificial Sequence
```

<220>
<223> DNA probe 2

<400> 6
cgaaatgatc ccatccagct gcgcgttgac cgc        33

<210> 7
<211> 33
<212> DNA
<213> Artificial Sequence

<220>
<223> DNA target 2

<400> 7
gcggtcaacg cgcagctgga tgggatcatt tcg        33

<210> 8
<211> 33
<212> DNA
<213> Artificial Sequence

<220>
<223> methyl DNA target 4 (C2)

<220>
<221> modified_base
<222> (2)..(2)
<223> m5c

<400> 8
gcggtcaacg cgcagctgga tgggatcatt tcg        33

<210> 9
<211> 33
<212> DNA
<213> Artificial Sequence

<220>
<223> methyl DNA target 5 (C2,16)

<220>
<221> modified_base
<222> (2)..(2)
<223> m5c

<220>
<221> modified_base
<222> (16)..(16)
<223> m5c

<400> 9
gcggtcaacg cgcagctgga tgggatcatt tcg        33

<210> 10
<211> 33
<212> DNA
<213> Artificial Sequence

```
<220>
<223> methyl DNA target 6 (C2,16,32)


<220>
<221> modified_base
<222> (2)..(2)
<223> m5c


<220>
<221> modified_base
<222> (16)..(16)
<223> m5c


<220>
<221> modified_base
<222> (32)..(32)
<223> m5c


<400> 10
gcggtcaacg cgcagctgga tgggatcatt tcg          33


<210> 11
<211> 48
<212> DNA
<213> Artificial Sequence


<220>
<223> methyl DNA target 7 (C39)


<220>
<221> modified_base
<222> (39) .. (39)
<223> m5c


<400> 11
gcggtcaacg cgcagctgga tgggatcatt tcggacttcg aaggtggg          48


<210> 12
<211> 33
<212> DNA
<213> Artificial Sequence


<220>
<223> N-DNA probe


<220>
<221> misc_difference
<222> (1)..(1)
<223> n represents electrically neutral c


<220>
<221> misc_difference
<222> (4)..(4)
<223> n represents electrically neutral a


<220>
<221> misc_difference
<222> (7)..(7)
```

<223> n represents electrically neutral      g

<220>
<221> misc_difference
<222> (10)..(10)
<223> n represents electrically neutral c

<220>
<221> misc_difference
<222> (13)..(13)
<223> n represents electrically neutral a

<220>
<221> misc_difference
<222> (16)..(16)
<223> n represents electrically neutral c

<400> 12
nganatnatn ccntcnagct gcgcgttgac cgc      33